Europäisches Patentamt

European Patent Office

Office européen des brevets

(19)

(11) **EP 1 351 188 A1**

(12) **EUROPEAN PATENT APPLICATION**
published in accordance with Art. 158(3) EPC

(43) Date of publication:
08.10.2003 Bulletin 2003/41

(51) Int Cl.⁷: **G06T 1/40**, G06N 3/02

(21) Application number: **01270860.8**

(86) International application number:
**PCT/ES01/00458**

(22) Date of filing: **27.11.2001**

(87) International publication number:
**WO 02/048963 (20.06.2002 Gazette 2002/25)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **14.12.2000 ES 200002992**

(71) Applicant: **Alma Bioinformatica SL**
**28760 Tres CAntos, Madrid (ES)**

(72) Inventor: **DOPAZO BLAZQUEZ, Joaquin,**
**Alma Bioinformatics, SL**
**E-28760 Tres Cantos (Madrid) (ES)**

(74) Representative: **Gomez-Acebo, Ignacio**
**Jorge Juan, 19 - 3.o Dcha**
**28001 Madrid (ES)**

(54) **GENE EXPRESSION DATA SELF-ORGANIZING ALGORITHM**

(57) A self-organizing algorithm for the massive analysis of gene expression data from DNA array experiments, beginning from a structure composed of two "daughter" neurons connected to a "mother" neuron, which are composed of a list of values or profile of initially random data. The algorithm divides the data set into data sub-sets in a successive series of cycles. Each of the cycles has a series of stages in which the data is introduced to the terminal neurons and the latter are updated. When the updating produced between two consecutive stages is below a certain pre-established minimum level, it is considered that the network has converged in that cycle and it is decided it the network should continue to grow or if it has already stopped growing, in which case the algorithm process is finalized.

FIG. 1

## Description

## OBJECT OF THE INVENTION

The present invention refers to a self-organizing algorithm of gene expression data on the basis of which it is possible to implement a process for the massive analysis of gene expression data from DNA array experiments, providing essential novelty features and significant advantages with respect to the known processes in the current State of the Art and intended for these same purposes.

More specifically, the invention proposes the development of a process beginning from a hierarchical structure composed of two "daughter" neurons connected to a "mother" neuron, which are composed of a list of values or profile, initially of random data. In a series of successive cycles, the algorithm divides the data set into sub-sets. Each of the cycles has a series of stages in which the sub-set data is introduced to the terminal neurons and the latter are updated. When the updating produced between two consecutive stages is below a certain pre-established minimum level, it is considered that the network has converged in that cycle and it is decided if the network should continue to grow, adding neurons in lower levels, or if it has stopped growing, in which case the algorithm process finalizes.

The field of application of the present invention is comprised within the biomedicine or bioscience industry, as well as within the food and agriculture sectors.

## BACKGROUND AND SUMMARY OF THE INVENTION

Currently, one of the largest problems associated with the use of DNA array techniques is the enormous volume of generated data.

The development of a SOTA (Self Organizing Tree Algorithm), (Dopazo and Carazo, 1997) neuronal network is known, originally designed for sorting sequences.

The process object of the present invention is based on said neuronal network but adapted to the massive analysis of gene expression data.

Specifically, the process is capable of finding clusters in which the gene expression is similar and separating them from those showing different expression patterns. The divisive form in which it functions implies that it divides the data set into sub-sets until defining the clusters with a similar expression. In addition, it does so hierarchically, in other words, the cluster groups are defined as a hierarchy of likenesses in which the most similar clusters are clustered into larger clusters and so on and so forth until describing the complete data set as a binary tree (or with multi-branches if it were necessary).

In this way, the self-organizing algorithm object of the invention achieves comparing the expression levels of different genes under different conditions such as temperature, a compound dose, patient tissue, etc.

Each DNA array, representing a condition, contains measures corresponding to the expression level of a series of genes under study (see Eisen et al, 1998 for details of the experiment). In the self-organizing algorithm, the gene expression profile or pattern is the list of expression values obtained in the different conditions (in other words, from each array). To facilitate the comparison between profiles, some mathematical transformation (logarithm and normalization or the like) is usually performed. Said comparison between profiles is done by means of a distance function. The most widely used are Euclidean distances (the sums of the absolute values of the item to item differences between the profile conditions) or correlations (measuring the likeness of the profile tendencies). The distance function is simply an objective way to describe the difference magnitude between two expression profiles.

More specifically, the algorithm begins from a structure composed of two "daughter" neurons connected to a "mother" neuron. Each of said neurons is a list of values or profile. Initially, the neurons are a collection of random numbers. The algorithm divides the data set into sub-sets in a successive series of cycles. Each of the cycles has a series of stages in which the data is introduced to the terminal neurons and the latter are updated. A stage consists introducing the entire data set to the terminal neurons. As the stages elapse, the neurons become updated, being set to mean values obtained from the data set. When the updating produced between two consecutive stages is below a certain pre-established minimum level, it is considered that the network has converged in that cycle and it is decided if the network should continue to grow or if it has stopped growing, in which case the algorithm process finalizes.

## BRIEF DESCRIPTION OF THE DRAWINGS

Other features and advantages of the invention will be clearly shown based on the following description of an embodiment example, carried out in a non-limiting and illustrative manner, making reference to the attached drawings in which:

Figure 1 shows the flow chart of the self-organizing algorithm object of the invention
Figure 2 schematically shows the adaptation and propagation manner of the likeness of the value of the neurons comprising the hierarchical structure of the network obtained by the algorithm of the previous figure.
Figure 3 shows a scheme of the updating manner and proximity between neurons.

## PREFERRED EMBODIMENT OF THE INVENTION

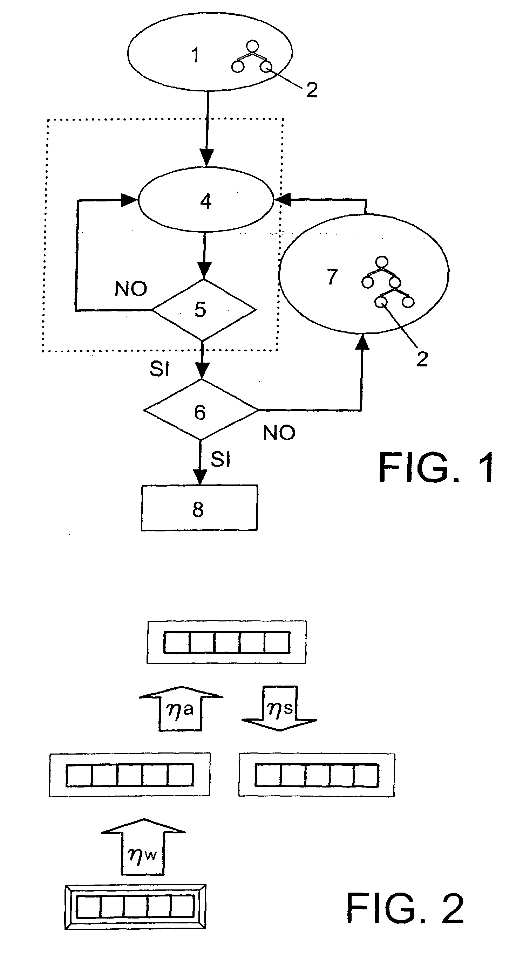

To carry out the detailed description of the preferred embodiment of the invention, permanent reference will be made to the drawings, in which figure 1 shows the flow chart of the self-organizing algorithm, which begins (1) from a structure composed of two "daughter" neurons connected to a "mother" neuron, each of said neurons being a list of values or profile (2), which are initially a collection of random numbers.

The next step consists of dividing the data set into data sub-sets in a series of successive cycles (3) in the following manner:

Each of the cycles (3) consists of a series of stages (4) in which the data is introduced to the terminal neurons (2) and the latter are updated.

A stage (4) consists of introducing the entire data set to the terminal neurons (2). In this way, as the stages elapse (4), the neurons become updated, being set to mean values obtained from the data set.

Between two consecutive stages (4), the convergence condition (5) of the cycle is checked, such that if the updating produced between two stages (4) is below a certain pre-established minimum level, it is considered that the network has converged in that cycle.

Next, after the cycle convergence, the convergence condition (6) of the network is checked, such that it is decided if the network should continue to grow, adding (7) a neuron and again initializing the cycle (3), or if it has already stopped growing, in which case the algorithm process is finalized (8).

The neuron updating is performed by using the relative network error. This is obtained from the resource:

$$R = \frac{\sum_{k=1}^{K} d_{P_k C_l}}{K}$$

which is the mean of the distance values between each profile and its corresponding winning neuron (2). The error is defined as:

$$E = \left| \frac{R_l - R_{l-1}}{R_{l-1}} \right| < \text{Threshold}$$

In other words, the relative increment in the resource value. When a significant drop in the resource value R is not achieved, it is considered that at the level of this cycle (3) the network has converged.

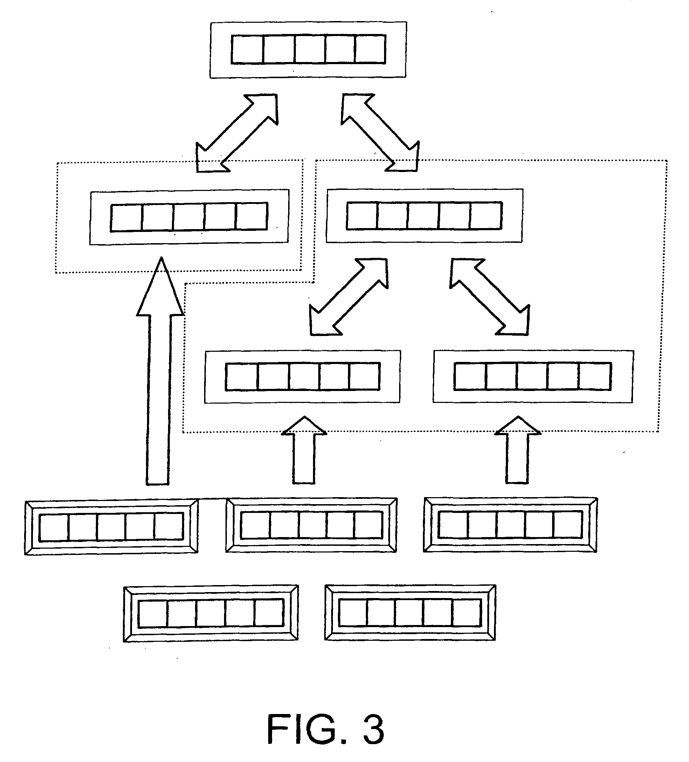

As has been explained, a stage (4) consists of introducing the entire data set to the terminal neurons (2), each introduction consisting of two steps: first, finding the neuron most similar to the profile being presented (winning neuron) among the terminal neurons, in other words, the neuron at the shortest distance to it, and next, the neuron and its neighborhood are updated, such as is shown in figure 3.

To prevent asymmetrical updating, the neighborhoods are defined as shown in figure 3. If it were not done in this manner, the upper neuron in the example of said figure would be updated by its daughter neuron on the left but not by the one on the right, which already has two descendents.

The updating process consists of these neurons modifying their values by means of the following formula (Kohonen, 1990):

$$C_i(\tau+1) = C_i(\tau) + \eta \cdot (P_j - C_i(\tau))$$

where n is the magnitude factor of the updating of the i neuron dependent on its to the "winning" neuron within the neighborhood; $C_i(t)$ is the i-th neuron in the introduction number $t_i$, and $P_j$ is the j-th expression profile.

In this manner, the neurons adapt their values to a mean value representative of the profile cluster associated to it. This value is very interesting because it represents the mean profile. At the same time, they propagate this likeness through the network as shown in figure 2 with n decreasing values.

Lastly, it is worth mentioning the possibility of stopping the network growth by means of a threshold for the resources the user can set, either it is possible to define a criteria based on the data in order to decide in which cycle (3) the network stops growing. For this, the original profiles are taken and random permutations are carried out on each of them in the order of the expression values. In this way, the correlation existing between profiles of the different genes is destroyed. Then, the distance values for each profile pair are calculated, and the distributions of values randomly. observed is shown. From this distribution, a distance value can be found, appearing with a very low probability simply randomly: for example, the value randomly observed in the 99% to 99.9% percentile. If the network growth stops when all the local resources are below the set distance value, we have profile clusters having a significantly higher likeness between than what would be expected randomly.

It is not necessary to extend the content of this description so that an expert in the matter can understand its scope and the advantages derived from the invention, as well as developing and putting into practice the object thereof.

However, its must be understood that the invention has been described according to one preferred embodiment thereof, due to which it is susceptible to modifications without implying any alteration whatsoever to its scope.

## Claims

1. A self-organizing algorithm for the massive analysis of gene expression data from DNA array experiments, based on the development of a SOTA (Self Organizing Tree Algorithm) neuronal network, **characterized in that** it is developed in the following manner:

- it begins (1) from a structure composed of two "daughter" neurons connected to a "mother" neuron, each of said neurons initially being a list of random values or random profile,
- the data set is divided into subsets in a successive series of cycles (3), each cycle (3) having a series of stages (4) in which the data is introduced to the terminal neurons (2) and the latter are updated, being set to mean values obtained from the data set,
- the convergence condition (5) of the cycle is checked between two consecutive stages, such that if the updating produced between two stages (4) is below a certain pre-established minimum level, it is considered that the network has converged **in that** cycle,
- likewise, the convergence condition (6) of the network is then checked, such that it is decided if the network should continue to grow, adding (7) a neuron and again initializing the cycle (3), or if it has already stopped growing, in which case the algorithm process is finalized (8).

2. A self-organizing algorithm according to claim 1, **characterized in that** the neuron updating is carried out by using the relative network error, or also called the relative increment in the resource value R, which is the measurement of the distance values between each profile and its corresponding winning neuron, such that when significant drop in the resource value R is not achieved, it is considered that the network has converged at the level of this cycle (3).

3. A self-organizing algorithm according to claims 1 and 2, **characterized in that** the updating process consists of the neurons modifying their values by means of the Kohonen formula:

$$C_i(\tau+1) = C_i(\tau)+\eta.(P_j - C_i(\tau))$$

where $\eta$ is the magnitude factor of the update of the i neuron dependent on its proximity to the "winning" neuron within the neighborhood; $C_i(t)$ is the i...$n$ neuron in the presentation number $t_i$, and $P_j$ is the $j$...$n$ expression profile.

4. A self-organizing algorithm according to the previous claims, **characterized in that** it is able to stop the network growth either by means of a threshold for the resources, possibly set by the user or by defining a criteria based on the data in order to decide in which cycle (3) the network stops growing, taking the original profiles and carrying out random permutations on each of them in the order of their expression values, thus destroying the correlation existing between the profiles of the different genes, and calculating the distance values for each profile pair in order to represent the distribution of values randomly observed, such that if the network growth stops when all the local resources are below the set distance value, profile clusters are obtained having a significantly higher likeness between than what would be expected randomly.

FIG. 1

FIG. 2

FIG. 3

**INTERNATIONAL SEARCH REPORT**

| International application No. |
|---|
| PCT/ES/01/00458 |

| A. | CLASSIFICATION OF SUBJECT MATTER |
|---|---|

**IPC 7** G06T 1/40, G06N 3/02

According to International Patent Classification (IPC) or to both national classification and IPC

| B. | FIELDS SEARCHED |
|---|---|

Minimum documentation searched (classification system followed by classification symbols)

**IPC 7** G06T 1/00, G06N3/00

Documentation searched other than minimum documentation to the extent that such documents are included in the fields searched

Electronic data base consulted during the international search (name of data base and, where practicable, search terms used)

**EPODOC, WPI, PAJ, CIBEPAT**

| C. | DOCUMENTS CONSIDERED TO BE RELEVANT |
|---|---|

| Category* | Citation of document, with indication, where appropriate, of the relevant passages | Relevant to claim No. |
|---|---|---|
| X | US 5933818 A (KASRAVI et al.) 03.08.1999, abstract; column 8-13. | 1-4 |
| Y | US 6035057 A (HOFFMAN) 07.03.2000, the whole document. | 1-4 |
| Y | EP 0410805 A2 (TOSHIBA KK.) 30.01.1991, the whole document. | 1-4 |
| X | US 5809490 A (GUIVER et al.) 15.09.1998, column 7, line 46- column 9, line 64; figure 6. | 1-4 |
| Y | EP 0845720 A1 (ABB PATENT GmbH.) 03.06.1998, the whole document. | 1-4 |
| Y | US 5729662 A (ROZMUS) 17.03.1998, the whole document. | 1-4 |
| A | EP 1037158 A2 (WHITEHEAD INSTITUTE FOR MEDICAL RESEARCH) 20.09.2000, the whole document. | 1-4 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **5 february 2002** | **15 february 2002** |

| Name and mailing address of the ISA/ | Authorized officer |
|---|---|
| **S.P.T.O.** | |
| Facsimile No. | Telephone No. |

Form PCT/ISA/210 (second sheet) (July 1992)

International Application No

*PCT/ES/01/00458*

| Patent document cited in search report | Publication date | Patent familiy member(s) | Publication date |
|---|---|---|---|
| EP 0845720 A1 | 03.06.1998 | DE19649633 A<br>DE59700716D D<br>US6321216 B | 04.06.1998<br>23.12.1999<br>20.11.2001 |
| EP 0410805 A2 | 30.01.1991 | JP3059757 A<br>US5274745 A | 14.03.1991<br>28.12.1993 |
| EP 1037158 A2 | 20.09.2000 | JP2000342299 A | 12.12.2000 |
| US 5809490 A | 15.09.1998 | NONE | |
| US 5933818 A | 03.08.1999 | NONE | |
| US 5729662 A | 17.03.1998 | NONE | |
| US 6035057 A | 07.03.2000 | CA2199588 A<br>US6278799 B | 10.09.1998<br>21.08.2001 |

Form PCT/ISA/210 (patent family annex) (July 1992)